(19)

Europäisches
Patentamt
European
Patent Office
Office européen
des brevets

(11) **EP 2 862 628 A1**

(12) **EUROPEAN PATENT APPLICATION**
published in accordance with Art. 153(4) EPC

(43) Date of publication:
**22.04.2015 Bulletin 2015/17**

(21) Application number: **13803691.8**

(22) Date of filing: **06.06.2013**

(51) Int Cl.:
***B01J 31/02*** (2006.01)     ***B01J 31/06*** (2006.01)
***C07C 9/04*** (2006.01)     ***C07C 1/12*** (2006.01)

(86) International application number:
**PCT/CN2013/076858**

(87) International publication number:
**WO 2013/185559 (19.12.2013 Gazette 2013/51)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **15.06.2012   CN 201210196937**

(71) Applicant: **Wuhan Kaidi Engineering Technology
Research Institute Co., Ltd.
Wuhan, Hubei 430212 (CN)**

(72) Inventors:
• **ZHANG, Yanfeng**
  **Wuhan**
  **Hubei 430223 (CN)**
• **ZHAN, Xiaodong**
  **Wuhan**
  **Hubei 430223 (CN)**

• **ZHENG, Xingcai**
  **Wuhan**
  **Hubei 430223 (CN)**
• **WANG, Zhilong**
  **Wuhan**
  **Hubei 430223 (CN)**
• **FANG, Zhangjian**
  **Wuhan**
  **Hubei 430223 (CN)**
• **XUE, Yongjie**
  **Wuhan**
  **Hubei 430223 (CN)**
• **TAO, Leiming**
  **Wuhan**
  **Hubei 430223 (CN)**

(74) Representative: **Zeuner Summerer Stütz
Nußbaumstrasse 8
80336 München (DE)**

(54) **LIQUID PHASE CO2 METHANATION CATALYST, PREPARATION METHOD AND USE THEREOF**

(57)     A liquid phase $CO_2$ methanation catalyst comprises amphipathic ionic liquid and metal active components dispersed in the amphipathic ionic liquid. The metal active components are in the state of stable colloid in the amphipathic ionic liquid, particle size is 0.5 to 20nm, and are in the shape of spherical. The metal active components include a first metal active component and a second metal active component. The first metal active component is nickel, the second metal active component is one or more of lanthanum, cerium, molybdenum, ruthenium, ytterbium, rhodium, palladium, platinum, potassium and magnesium, and the molar ratio of the first metal active component to the second metal active component is 10:0.1-2. Also provided are a preparation method of the liquid phase $CO_2$ methanation catalyst and a use for $CO_2$ methanation thereof.

**FIG. 1**

**EP 2 862 628 A1**

**Description**

FIELD OF THE INVENTION

The invention relates to a liquid catalyst for methanation of carbon dioxide.

BACKGROUND OF THE INVENTION

Natural gas is a highquality, clean, high efficiency hydrocarbon resource. With the development of urbanization and the improvement of people's living standard, demand for natural gas is increased quickly. The supply of natural gas is insufficient. The synthesis of natural gas is an effective method for transforming high carbon energy to a lowcarbon and hydrogen-rich energy, with good environmental benefits and economic benefits.

In recent years, increased $CO_2$ emission leads to global warming. $CO_2$ methanation can not only reduce $CO_2$ emission, but also can synthesize high quality and clean energy. Thus, $CO_2$ methanation reaction has attracted widespread attention. In the past few decades, Ni based catalyst, which is regarded as a methanation non noble metal catalyst having the highest activity, has been widely studied. Chinese Patent Publication No. CN102091629A and No. CN101773833A disclose a nickelbased $CO_2$ methanation catalyst. The preparation method of the catalyst consumes large energy, and the activity of the catalyst decreases greatly under high temperature and high water vapor partial pressure. The catalyst layer deposited on the surface of the substrate tends to erode and fall off, which greatly limits the application of the $CO_2$ methanation catalyst.

$CO_2$ methanation reaction releases strong heat energy and low temperature can accelerate the reaction. Based on the theory and combined with a unique structure and confinement effect of a metal nanoparticle agent, the invention provides a liquid catalyst for methanation of $CO_2$ under liquid phase and low temperature conditions.

SUMMARY OF THE INVENTION

In view of the abovedescribed problems, it is one objective of the invention to provide a liquid catalyst for methanation of carbon dioxide under liquid phase and low temperature conditions, and apreparation method and use thereof.

To achieve the above objective, in accordance with one embodiment of the invention, there is provided a liquid catalyst for methanation of carbon dioxide, comprising an amphiphilic ionic liquid and a metal active component dispersed in the amphiphilic ionic liquid. The metal active component is dispersed in the amphiphilic ionic liquid in the form of stable colloid, and the colloid is spherical and has a particle size of between 0.5 and 20 nm. The metal active component comprises a first metal active component and a second metal active component. The first metal active component comprises nickel, and the second metal active component is selected from the group consisting of lanthanum, cerium, molybdenum, ruthenium, ytterbium, rhodium, palladium, platinum, potassium, magnesium, or a mixture thereof. A molar ratio of the first metal active component to the second metal active component is between 10: 0.1 and 10: 2.

In a class of this embodiment, a molar ratio of the amphiphilic ionic liquid to the first metal active component is between 10: 0.1 and 10: 2.

In a class of this embodiment, the amphiphilic ionic liquid is an amphiphilic small molecular ionic liquid or an amphiphilic polymer ionic liquid; the amphiphilic small molecular ionic liquid comprises N pyridinium hydrochloride, N pyridinium tetrafluoroborate, 1,3 tetrafluoroborate, 1,3hydrochloride, 1,3 hexafluorophosphate, and the alkyl has a carbon chain length of between 8 and 18; the amphiphilic polymer ionic liquid comprises poly(4), poly(4)(trifluoromethyl)sulfonylimide, poly(4) tetrafluoroborate, poly(4) tetrafluoroborate, poly(4) polypyrrolidone hydrochloride.

In accordance with another embodiment of the invention, there provided is a method for preparation of a liquid catalyst for methanation of carbon dioxide, the method comprising:

1) dispersing a soluble salt of a metal active component and an amphiphilic ionic liquid in a liquid medium to yield a mixed solution, the metal active component comprising a first metal active component and a second metal active component with a molar ratio of metal ions thereof being between 10: 0.1 and 10: 2;

2) regulating a pH value of the mixed solution to 8, stirring for between 1 and 3 hours, charging hydrogen to reduce the mixed solution at a temperature of between 100 and 200°C and a pressure of between 0.1 and 4.0 MPa for between 1 and 6 hours; and

3) centrifuging, filtering, washing, and drying a resulting product.

In a class of this embodiment, the soluble salt of the first metal active component is a nitrate, acetate, or chloride of nickel, and the soluble salt of the second metal active component is a nitrate thereof.

In a class of this embodiment, a molar ratio of the amphiphilic ionic liquid to metal ions of the soluble salt of the first metal active component is between 10: 0.1 and 10: 2, and a total concentration of the metal ions in the mixed solution is between 0.0001 and 0.01 mol/L.

In a class of this embodiment, the liquid medium comprises water, alcohols, tetrahydrofuran, acetonitrile, 1,4, nhexane, and cyclohexane.

The invention further provides a method for methanation of carbon dioxide, the method comprising adding to a reactor the liquid catalyst and a solvent, controlling a concentration of the liquid catalyst in a reaction system to be between 0.0001 and 0.01 mol/L, charging hydrogen and carbon dioxide to the reactor, heating the reactor to be between 100 and 120°C, and allowing the hydrogen and the carbon dioxide to react at a pressure of between 0.1 and 4.0 MPa for between 1 and 6 hours.

In a class of this embodiment, the solvent comprises water, alcohols, tetrahydrofuran, acetonitrile, 1,4, n, and cyclohexane.

In a class of this embodiment, a molar ratio of the hydrogen to the carbon dioxide is between 0.5: 1 and 5: 1, preferably, 4.0. The catalytic pressure is preferably at 3 MPa.

$CO_2$ methanation reaction releases strong heat energy and low temperature can accelerate the reaction. Based on the theory and taking into account a unique structure and confinement effect of a metal nanoparticle agent, the invention provides a liquid catalyst for methanation of $CO_2$ under liquid phase and low temperature conditions. The catalytic activity, methane selectivity and stability of the catalyst are all controllable.

Advantages according to embodiments of the invention are summarized as follows:

1. The metal active component of the $CO_2$ methanation catalyst has small particle size (0.5nm) and narrow particle size distribution, and is uniformly dispersed in the amphiphilic ionic liquid to form stable collide. No coagulation occurs before reaction. After reaction, the catalyst is easy to separate from the products such as methane and reaction mediums, which is beneficial to the recycling of the catalyst.

2. The amphiphilic ionic liquid can prevent the coagulation of the metal active nanoparticles, facilitate $CO_2$ to adsorb on the surface of the metal active nanoparticles, enhance the $CO_2$ concentration in the active site, and facilitate the $CO_2$ methanation. The $CO_2$ methanation can be achieved at liquid state and 140°C. The catalyst has good low temperature catalytic activity, methane selectivity, and good thermal stability. The common problems such as falling off of solid catalysts at high temperature and the coagulation of the active components are avoided. The application of the catalyst can save the energy consumption and reduce the production costs.

3. The preparation method of the catalyst is simple, has low costs, and is suitable for popularization.

BRIEF DESCRIPTION OF THE DRAWINGS

FIG. 1 is a SEM of a liquid catalyst for methanation of carbon dioxide prepared in Example 1; and

FIG. 2 is a diagram showing the activity of a liquid catalyst for methanation of carbon dioxide after 300 hours' methanation reaction.

DETAILED DESCRIPTION OF THE EMBODIMENTS

For further illustrating the invention, experiments detailing a liquid catalyst for methanation of carbon dioxide are described below. It should be noted that the following examples are intended to describe and not to limit the invention.

**Example 1**

Nickel nitrate, magnesium nitrate, and an ionic liquid of 1hexadecyl-3 methylimidazole hydrochloride were mixed according to a molar ratio thereof of 1: 0.2: 20 and dispersed in a liquid medium, for example, water, to yield a mixed solution. In the mixed solution, the molar concentration of nickel nitrate was $2.79 \times 10^{-4}$ mol/L. The pH value of the mixed solution was adjusted using aqueous ammonia to 9.0. The mixed solution was stirred for 3 hours and transferred to a reactor. The reactor was heated to 150°C and aerated with hydrogen at the pressure of 3.0 MPa for2 hours. The resulting product was centrifuged, filtered, washed respectively with water and alcohol, and dried. TEM measurement showed that, the nanoparticles of the metal active component of the liquid catalyst for methanation of carbon dioxide had an average particle size of 11.8 nm, with a narrow and uniform distribution.

The liquid catalyst is applied to methanation of $CO_2$. Specifically, the liquid catalyst and water were added to a reactor. The concentration of the liquid catalyst in the reaction system was controlled at 0.005 mol/L. $CO_2$ and $H_2$ were

charged to the reactor according to the equation $nH_2: nCO_2=4$, heated to 150°C, and allowed to react for 2 hours at the pressure of 3 MPa. The $CO_2$ conversion rate and methane selectivity were listed in Table **1.**

**Example 2**

Nickel acetate, lanthanum nitrate, and an ionic liquid of loctadecyl-3 methylimidazolehexafluorophosphate were mixed according to a molar ratio thereof of 1: 0.15: 20 and dispersed in a liquid medium, for example, alcohol, to yield a mixed solution. In the mixed solution, the molar concentration of nickel acetate was $3.61 \times 10^{-4}$ mol/L. The pH value of the mixed solution was adjusted using triethylamine to 8.0. The mixed solution was stirred for 2 hours and transferred to a reactor. The reactor was heated to 100°C and aerated with hydrogen at the pressure of 3.0 MPa for 1.5 hours. The resulting product was centrifuged, filtered, washed respectively with water and alcohol, and dried.
The liquid catalyst is applied to methanation of $CO_2$. Specifically, the liquid catalyst and water were added to a reactor. The concentration of the liquid catalyst in the reaction system was controlled at 0.005 mol/L. $CO_2$ and $H_2$ were charged to the reactor according to the equation $nH_2: nCO_2=4$, heated to 150°C, and allowed to react for 2 hours at the pressure of 3 MPa. The $CO_2$, conversion rate and methane selectivity were listed in Table **1.**

**Example 3**

Nickel nitrate, cerium nitrate, and an ionic liquid of N dodecyl pyridinium tetrafluoroborate were mixed according to a molar ratio thereof of 1: 0.15: 18 and dispersed in a liquid medium, for example, acetonitrile, to yield a mixed solution. In the mixed solution, the molar concentration of nickel nitrate was $1.32x\ 10^{-3}$ mol/L. The pH value of the mixed solution was adjusted using diisopropyl tertbutylamine to 10.0. The mixed solution was stirred for 3 hours and transferred to a reactor. The reactor was heated to 150°C and aerated with hydrogen at the pressure of 3.0 MPa for 1.5 hours. The resulting product was centrifuged, filtered, washed respectively with water and alcohol, and dried.
The liquid catalyst is applied to methanation of $CO_2$. Specifically, the liquid catalyst and water were added to a reactor. The concentration of the liquid catalyst in the reaction system was controlled at 0.005 mol/L. $CO_2$ and $H_2$ were charged to the reactor according to the equation $nH_2: nCO_2=4$, heated to 120°C, and allowed to react for 3 hours at the pressure of 1 MPa. The $CO_2$ conversion rate and methane selectivity were listed in Table 1.

**Example 4**

Nickel nitrate, molybdenum nitrate, and an ionic liquid of poly 1(4 styryl) 3 methylimidazole(trifluoromethyl)sulfonylimide were mixed according to a molar ratio thereof of 1: 0.12: 16 and dispersed in a liquid medium, for example, water, to yield a mixed solution. In the mixed solution, the molar concentration of nickel nitrate was $6.35 \times 10^{-4}$ mol/L. The pH value of the mixed solution was adjusted using aqueous ammonia to 10.0. The mixed solution was stirred for 2.5 hours and transferred to a reactor. The reactor was heated to 200°C and aerated with hydrogen at the pressure of 2.0 MPa for 2 hours. The resulting product was centrifuged, filtered, washed respectively with water and alcohol, and dried.
The liquid catalyst is applied to methanation of $CO_2$. Specifically, the liquid catalyst and dioxane were added to a reactor. The concentration of the liquid catalyst in the reaction system was controlled at 0.005 mol/L. $CO_2$ and $H_2$ were charged to the reactor according to the equation $nH_2: nCO_2=4$, heated to 150°C, and allowed to react for one hour at the pressure of 13 MPa. The $CO_2$ conversion rate and methane selectivity were listed in Table 1.

**Example 5**

Nickel nitrate, ruthenium nitrate, potassium nitrate, and an ionic liquid of N hexadecylpyridinium tetrafluoroborate were mixed according to a molar ratio thereof of 1: 0.1; 0.1: 20 and dispersed in a liquid medium, for example, tetrahydrofuran, to yield a mixed solution. In the mixed solution, the molar concentration of nickel nitrate was $8.1 \times 10^{-4}$ mol/L. The pH value of the mixed solution was adjusted using aqueous ammonia to 8.0. The mixed solution was stirred for one hour and transferred to a reactor. The reactor was heated to 150°C and aerated with hydrogen at the pressure of 1.0 MPa for 5 hours. The resulting product was centrifuged, filtered, washed respectively with water and alcohol, and dried.
The liquid catalyst is applied to methanation of $CO_2$. Specifically, the liquid catalyst and tetrahydrofuran were added to a reactor. The concentration of the liquid catalyst in the reaction system was controlled at 0.005 mol/L. $CO_2$ and $H_2$ were charged to the reactor according to the equation $nH_2: nCO_2=4$, heated to 110°C, and allowed to react for 2.5 hours at the pressure of 3 MPa. The $CO_2$ conversion rate and methane selectivity were listed in Table **1.**

**Example 6**

Nickel nitrate, ytterbium nitrate, and an ionic liquid of poly 1(4 styryl) 3 methylimidazole tetrafluoroborate were mixed according to a molar ratio thereof of 1: 0.15: 15 and dispersed in a liquid medium, for example, cyclohexane, to yield a mixed solution. In the mixed solution, the molar concentration of nickel nitrate was $9.81 \times 10^{-4}$ mol/L. The pH value of the mixed solution was adjusted using aqueous ammonia to 8.0. The mixed solution was stirred for 2 hours and transferred to a reactor. The reactor was heated to 150°C and aerated with hydrogen at the pressure of 0.1 MPa for 3 hours. The resulting product was centrifuged, filtered, washed respectively with water and alcohol, and dried.

The liquid catalyst is applied to methanation of $CO_2$. Specifically, the liquid catalyst and cyclohexane were added to a reactor. The concentration of the liquid catalyst in the reaction system was controlled at 0.005 mol/L. $CO_2$ and $H_2$ were charged to the reactor according to the equation $nH_2$: $nCO_2$=0.5) heated to 190°C, and allowed to react for 1.5 hours at the pressure of 4 MPa. The $CO_2$ conversion rate and methane selectivity were listed in Table 1.

**Example 7**

Nickel nitrate, rhodium nitrate, and an ionic liquid of polyl(4 styryl) 3 butyllimidazole tetrafluoroborate were mixed according to a molar ratio thereof of 1; 0.16: 10 and dispersed in a liquid medium, for example, alcohol, to yield a mixed solution. In the mixed solution, the molar concentration of nickel nitrate was $1.28 \times 10^{-3}$ mol/L. The pH value of the mixed solution was adjusted using aqueous ammonia to 8.0. The mixed solution was stirred for 3 hours and transferred to a reactor. The reactor was heated to 100°C and aerated with hydrogen at the pressure of 0.5 MPa for 2 hours. The resulting product was centrifuged, filtered, washed respectively with water and alcohol, and dried.

The liquid catalyst is applied to methanation of $CO_2$. Specifically, the liquid catalyst and alcohol were added to a reactor. The concentration of the liquid catalyst in the reaction system was controlled at 0.003 mol/L. $CO_2$ and $H_2$ were charged to the reactor according to the equation $nH_2$, $nCO_2$=4, heated to 110°C, and allowed to react for 2 hours at the pressure of 3 MPa. The $CO_2$ conversion rate and methane selectivity were listed in Table **1.**

**Example 8**

Nickel nitrate, palladiumnitrate, and an ionic liquid of N octyl pyridinium tetrafluoroborate were mixed according to a molar ratio thereof of 1: 0.01: 100 and dispersed in a liquid medium, for example, dioxane, to yield a mixed solution. In the mixed solution, the molar concentration of nickel nitrate was $3.18 \times 10^{-3}$ mol/L. The pH value of the mixed solution was adjusted using aqueous ammonia to 8.0. The mixed solution was stirred for 3 hours and transferred to a reactor. The reactor was heated to 200°C and aerated with hydrogen at the pressure of 3.0 MPa for 2 hours. The resulting product was centrifuged, filtered, washed, and dried.

The liquid catalyst is applied to methanation of $CO_2$. Specifically, the liquid catalyst and dioxane were added to a reactor. The concentration of the liquid catalyst in the reaction system was controlled at 0.0005 mol/L. $CO_2$ and $H_2$ were charged to the reactor according to the equation $nH_2$: $nCO_2$=3, heated to 120°C, and allowed to react for 2 hours at the pressure of 3 MPa. The $CO_2$ conversion rate and methane selectivity were listed in Table 1.

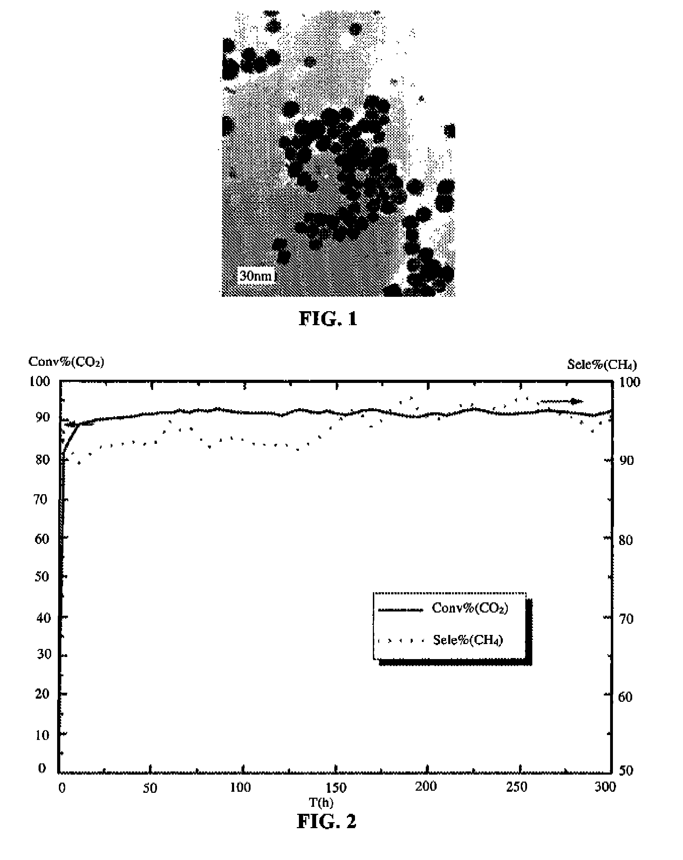

The catalytic reaction was carried out for consecutive 300 hours, and the $CO_2$ conversion rate and methane selectivity were measured along with the reaction time. As shown in FIG. 2, the liquid catalyst for methanation of carbon dioxide had good stability.

**Example 9**

Nickel nitrate, platinum nitrate, and an ionic liquid of poly 1(4 styryl) 3 butyllimidazole polypyrrolidone hydrochloride were mixed according to a molar ratio thereof of 1: 0.05: 5 and dispersed in a liquid medium, for example, nhexane, to yield a mixed solution. In the mixed solution, the molar concentration of nickel nitrate was $6.28 \times 10^{-4}$ mol/L. The pH value of the mixed solution was adjusted using aqueous ammonia to 8.0. The mixed solution was stirred for 3 hours and transferred to a reactor. The reactor was heated to 100°C and aerated with hydrogen at the pressure of 4.0 MPa for 2 hours. The resulting product was centrifuged, filtered, washed respectively with water and alcohol, and dried.

The liquid catalyst is applied to methanation of $CO_2$. Specifically, the liquid catalyst and water were added to a reactor. The concentration of the liquid catalyst in the reaction system was controlled at 0.00015 mol/L. $CO_2$ and $H_2$ were charged to the reactor according to the equation $nH_2$: $nCO_2$=5, heated to 200°C, and allowed to react for 2 hours at the pressure of 2 MPa. The $CO_2$ conversion rate and methane selectivity were listed in Table 1.

**Example 10**

Nickel nitrate, cerium nitrate, magnesium nitrate, and an ionic liquid of 1,3 dioctylimidazole tetrafluoroborate were mixed according to a molar ratio thereof of 1: 0.15: 0.05: 10 and dispersed in a liquid medium, for example, acetonitrile, to yield a mixed solution. In the mixed solution, the molar concentration of nickel nitrate was $3.18 \times 10^{-3}$ mol/L. The pH value of the mixed solution was adjusted using triethylamine to 9.0. The mixed solution was stirred for 3 hours and transferred to a reactor. The reactor was heated to 150°C and aerated with hydrogen at the pressure of 3.0 MPa for 2 hours. The resulting product was centrifuged, filtered, washed, and dried.

The liquid catalyst is applied to methanation of $CO_2$. Specifically, the liquid catalyst and n hexane were added to a reactor. The concentration of the liquid catalyst in the reaction system was controlled at 0.01 mol/L. $CO_2$ and $H_2$ were charged to the reactor according to the equation $nH_2$: $nCO_2$=4, heated to 100°C, and allowed to react for 2 hours at the pressure of 3 MPa. The $CO_2$ conversion rate and methane selectivity were listed in Table **1**.

The $CO_2$ conversion rate and methane selectivity are calculated according to the following equations:

$$CO_2 \text{ conversion rate} = (\text{mole number of reacted } CO_2/\text{total mole number of } CO_2 \text{ in raw gas}) \times 100\%$$

$$\text{Methane selectivity} = (\text{mole number of produced methane/total mole number of reacted } CO_2) \times 100\%$$

Table 1 $CO_2$ conversion rate and methane selectivity

| Example | Conv. % ($CO_2$) | Sele. % ($CH_4$) |
|---------|------------------|------------------|
| 1 | 88.3 | 93.5 |
| 2 | 80.6 | 92.5 |
| 3 | 86.7 | 89.3 |
| 4 | 73.2 | 91.2 |
| 5 | 85.2 | 81.3 |
| 6 | 75.9 | 84.6 |
| 7 | 62.8 | 89.5 |
| 8 | 65.7 | 75.3 |
| 9 | 80.6 | 86.9 |
| 10 | 59.3 | 93.1 |

**Claims**

1. A liquid catalyst for methanation of carbon dioxide, **characterized by** comprising an amphiphilic ionic liquid and a metal active component dispersed in the amphiphilic ionic liquid, wherein

   the metal active component is dispersed in the amphiphilic ionic liquid in the form of stable colloid, and the colloid is spherical and has a particle size of between 0.5 and 20 nm;
   the metal active component comprises a first metal active component and a second metal active component;
   the first metal active component comprises nickel, and the second metal active component is selected from the group consisting of lanthanum, cerium, molybdenum, ruthenium, ytterbium, rhodium, palladium, platinum, potassium, magnesium, or a mixture thereof; and
   a molar ratio of the first metal active component to the second metal active component is between 10: 0.1 and 10: 2.

2. The liquid catalyst of claim 1, **characterized in that** a molar ratio of the amphiphilic ionic liquid to the first metal active component is between 10: 0.1 and 10:2.

3. The liquid catalyst of claim 1, **characterized in that**

   the amphiphilic ionic liquid is an amphiphilic small molecular ionic liquid or an amphiphilic polymer ionic liquid;

the amphiphilic small molecular ionic liquid comprises N alkyl pyridinium hydrochloride, N alkyl pyridinium tetrafluoroborate, 1,3 dialkylimidazole tetrafluoroborate, 1,3 dialkylimidazole hydrochloride, 1,3 dialkylimidazole hexafluorophosphate, and the alkyl has a carbon chain length of between8 and 18;

the amphiphilic polymer ionic liquid comprises poly 1 (4 styryl) 3 methylimidazolehexafluorophosphate, poly1(4 styryl)3 methylimidazole(trifluoromethyl)sulfonylimide, poly1(4 styryl)3 methylimidazoletetrafluoroborate, poly1(4 styryl) 3butyllimidazole tetrafluoroborate, poly1(4 styryl) 3 butyllimidazole polypyrrolidone hydrochloride.

4. A method for preparation of a liquid catalyst for methanation of carbon dioxide, the method comprising:

1) dispersing a soluble salt of a metal active component and an amphiphilic ionic liquid in a liquid medium to yield a mixed solution, the metal active component comprising a first metal active component and a second metal active component with a molar ratio of metal ions thereof being between 10: 0.1 and 10: 2;

2) regulating a pH value of the mixed solution to 8-10, stirring for between 1 and 3 hours, charging hydrogen to reduce the mixed solution at a temperature of between 100 and 200°C and a pressure of between 0.1 and 4.0 MPa for between 1 and 6 hours; and

3) centrifuging, filtering, washing, and drying a resulting product.

5. The method of claim 4, **characterized in that** the soluble salt of the first metal active component is a nitrate, acetate, or chloride of nickel, and the soluble salt of the second metal active component is a nitrate thereof.

6. The method of claim 4, **characterized in that** a molar ratio of the amphiphilic ionic liquid to metal ions of the soluble salt of the first metal active component is between 10: 0.1 and 10: 2, and a total concentration of the metal ions in the mixed solution is between 0.0001 and 0.01 mol/L.

7. The method of claim 4, **characterized in that** the liquid medium comprises water, alcohols, tetrahydrofuran, acetonitrile, 1,4 dioxane, nhexane, and cyclohexane.

8. A method for methanation of carbon dioxide, the method comprising adding to a reactor the liquid catalyst of claim 1 and a solvent, controlling a concentration of the liquid catalyst in a reaction system to be between 0.0001 and 0.01 mol/L, charging hydrogen and carbon dioxide to the reactor, heating the reactor to be between 100 and 120°C, and allowing the hydrogen and the carbon dioxide to react at a pressure of between 0.1 and 4.0 MPa for between 1 and 6 hours.

9. The method of claim 8, **characterized in that** the solvent comprises water, alcohols, tetrahydrofuran, acetonitrile, 1,4 dioxane, nhexane, and cyclohexane.

10. The method of claim 8, **characterized in that** a molar ratio of the hydrogen to the carbon dioxide is between 0.5: 1 and 5:1.

**FIG. 1**

**FIG. 2**

# INTERNATIONAL SEARCH REPORT

| | International application No. |
|---|---|
| | PCT/CN2013/076858 |

**A. CLASSIFICATION OF SUBJECT MATTER**

See the extra sheet

According to International Patent Classification (IPC) or to both national classification and IPC

**B. FIELDS SEARCHED**

Minimum documentation searched (classification system followed by classification symbols)

IPC: B01J; C07C

Documentation searched other than minimum documentation to the extent that such documents are included in the fields searched

Electronic data base consulted during the international search (name of data base and, where practicable, search terms used)

CNPAT, CNKI, WPI, EPODOC: methanation, carbon dioxide, CO2, liquid phase, aqueous, ionic liquid, imidazole, poly pyrrolidone, gel, colloid, nanometer, nm, nickel, Ni

**C. DOCUMENTS CONSIDERED TO BE RELEVANT**

| Category* | Citation of document, with indication, where appropriate, of the relevant passages | Relevant to claim No. |
|---|---|---|
| P, X | CN 102716766 A (WUHAN KAIDI ENG TECHNOLOGY RESEARCH INSTITUTE CO LTD) 10 October 2012 (10.10.2012) claims 1 to 10 | 1-10 |
| P, Y | CN 103111308 A (PEKING UNIVERSITY) 22 May 2013 (22.05.2013) description, paragraphs [0005] to [0014] | 1-10 |
| Y | CN 101045206 A (PEKING UNIVERSITY) 03 October 2007 (03.10.2007) description, page 1, line23 to page 3, line 9 | 1-10 |
| Y | CN 102247850 A (XIAO, Tiancun) 23 November 2011 (23.11.2011) description, paragraphs [0011] to [0013], embodiment 1, page 7, lines 15 and 16 | 1-10 |
| A | GB 2463878 (DCA CONSULTANTS LTD) 31 March 2010 (31.03.2010) the whole document | 1-10 |

☐ Further documents are listed in the continuation of Box C.  ☒ See patent family annex.

| | |
|---|---|
| *    Special categories of cited documents:<br><br>"A"   document defining the general state of the art which is not considered to be of particular relevance<br><br>"E"   earlier application or patent but published on or after the international filing date<br><br>"L"   document which may throw doubts on priority claim(s) or which is cited to establish the publication date of another citation or other special reason (as specified)<br><br>"O"   document referring to an oral disclosure, use, exhibition or other means<br><br>"P"   document published prior to the international filing date but later than the priority date claimed | "T"   later document published after the international filing date or priority date and not in conflict with the application but cited to understand the principle or theory underlying the invention<br><br>"X"   document of particular relevance; the claimed invention cannot be considered novel or cannot be considered to involve an inventive step when the document is taken alone<br><br>"Y"   document of particular relevance; the claimed invention cannot be considered to involve an inventive step when the document is combined with one or more other such documents, such combination being obvious to a person skilled in the art<br><br>"&"document member of the same patent family |
| Date of the actual completion of the international search<br><br>01 August 2013 (01.08.2013) | Date of mailing of the international search report<br><br>19 September 2013 (19.09.2013) |
| Name and mailing address of the ISA<br>State Intellectual Property Office of the P. R. China<br>No. 6, Xitucheng Road, Jimenqiao<br>Haidian District, Beijing 100088, China<br>Facsimile No. (86-10) 62019451 | Authorized officer<br><br>PAN, Hui<br><br>Telephone No. (86-10) 62084769 |

Form PCT/ISA /210 (second sheet) (July 2009)

## INTERNATIONAL SEARCH REPORT
### Information on patent family members

| | International application No. |
| --- | --- |
| | PCT/CN2013/076858 |

| Patent Documents referred in the Report | Publication Date | Patent Family | Publication Date |
| --- | --- | --- | --- |
| CN 102716766 A | 10.10.2012 | None | |
| CN 103111308 A | 22.05.2013 | None | |
| CN 101045206 A | 03.10.2007 | CN 100493701 C | 03.06.2009 |
| CN 102247850 A | 23.11.2011 | CN 102247850 B | 26.06.2013 |
| GB 2463878 A | 31.03.2010 | GB 2463878 B | 21.11.2012 |

Form PCT/ISA/210 (patent family annex) (July 2009)

## INTERNATIONAL SEARCH REPORT

International application No.

PCT/CN2013/076858

Continuation of the second sheet : A. classification of subject matter

B01J 31/02 (2006.01) i

B01J 31/06 (2006.01) i

C07C 9/04 (2006.01) i

C07C 1/12 (2006.01) i

Form PCT/ISA /210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 102091629 A

- CN 101773833 A